Europäisches Patentamt
European Patent Office
Office européen des brevets

(19) (11) Publication number: **0 091 755**
**A1**

(12) # EUROPEAN PATENT APPLICATION

(21) Application number: 83301785.8

(22) Date of filing: 30.03.83

(51) Int. Cl.³: C 07 D 499/68
C 07 D 499/70, A 61 K 31/43

(30) Priority: 03.04.82 GB 8209981

(43) Date of publication of application:
19.10.83 Bulletin 83/42

(84) Designated Contracting States:
CH DE FR GB IT LI NL

(71) Applicant: BEECHAM GROUP PLC
Beecham House Great West Road
Brentford Middlesex(GB)

(72) Inventor: Best, Desmon John
2a Little Bridges Close
Southwater Sussex(GB)

(72) Inventor: Burton, George
14 Windborough Road
Carshalton Surrey(GB)

(72) Inventor: Harrington, Frank Peter
18 Epping Walk Furnace Green
Crawley Sussex(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **Penicillin derivatives, a process for their preparation and compositions containing them.**

(57) A compound of formula (I) or a pharmaceutically acceptable salt or *in vivo* hydrolysable ester thereof:

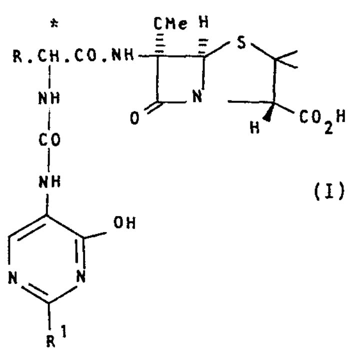

(I)

wherein R represents optionally substituted aryl or heterocyclyl; and $R^1$ represents hydrogen, an alkyl, alkenyl, cycloalkyl or cycloalkenyl group, an optionally substituted phenyl, a cyclopropyl alkyl group, a hydroxy group optionally substituted by alkyl, alkenyl, cycloalkyl, phenyl or benzyl, an optionally substituted mercapto group, an alkylsulphenyl group, a free or substituted amino group, an optionally substituted piperazino or phenyl- alkylamino group, an acylamino group or an alkyl, aralkyl or arylsulphonyl amino group; a process for the preparation of such compounds and pharmaceutical compositions comprising them.



Croydon Printing Company Ltd.

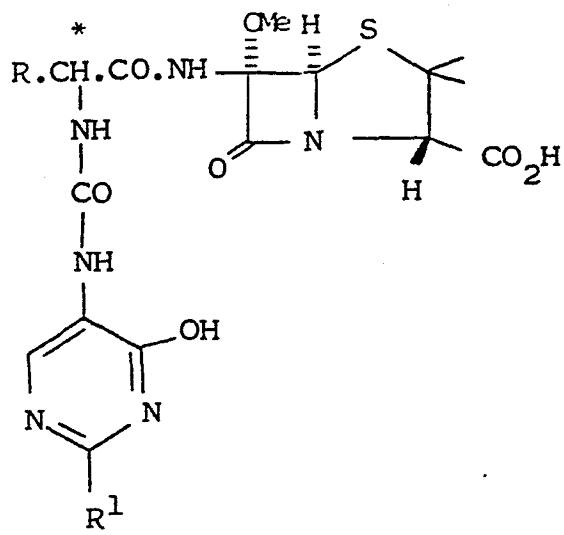

(I)

wherein R represents optionally substituted aryl or heterocyclyl; and $R^1$ represents hydrogen, an alkyl, alkenyl, cycloalkyl or cycloalkenyl group, an optionally substituted phenyl, a cyclopropyl alkyl group, a hydroxy group optionally substituted by alkyl, alkenyl, cycloalkyl, phenyl or benzyl, an optionally substituted mercapto group, an alkylsulphenyl group, a free or substituted amino group, an optionally substituted piperazino or phenyl- alkylamino group, an acylamino group or an alkyl, aralkyl or arylsulphonyl amino group.

The term "heterocyclyl" includes single or fused rings comprising up to four hetero atoms in the ring selected from oxygen, nitrogen and sulphur and optionally substituted with up to three halogen, $C_{1-6}$ alkyl, $C_{1-6}$ alkoxy, halo-($C_{1-6}$)-alkyl, hydroxy, amino, carboxy, $C_{1-6}$ alkoxycarbonyl, $C_{1-6}$ alkoxycarbonyl($C_{1-6}$) alkyl, aryl or oxo groups.

Suitably the heterocyclic ring comprises from 4 to 7 ring atoms, preferably 5 to 6 atoms.

When used herein the term "aryl" includes phenyl and naphthyl optionally substituted with up to five, preferably up to three, groups selected from halogen,

$C_{1-6}$ alkyl, phenyl, $C_{1-6}$ alkoxy, halo($C_{1-6}$) alkyl, hydroxy, amino, nitro, carboxy, $C_{1-6}$ alkoxycarbonyl, $C_{1-6}$ alkoxycarbonyl-($C_{1-6}$)-alkyl $C_{1-6}$ alkylcarbonyloxy, or $C_{1-6}$ alkylcarbonyl groups.

Examples of suitable pharmaceutically acceptable in vivo hydrolysable ester groups include those which break down readily in the human body to leave the parent acid or its salts, for example acyloxyalkyl groups, such as acetoxymethyl, pivaloyloxymethyl, α-acetoxyethyl and α-pivaloyloxyethyl groups; alkoxycarbonyloxyalkyl groups, such as ethoxycarbonyloxymethyl and α-ethoxycarbonyloxyethyl; dialkylaminoalkyl groups, such as dimethylaminomethyl, dimethylaminoethyl, diethylaminomethyl or diethylaminoethyl; and lactone groups such as phthalidyl or dimethoxyphthalidyl.

Suitable pharmaceutically acceptable salts of the compounds of formula (I) include metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N'-bisdehydroabietylamine, ethylenediamine, or bases of the pyridine type such as pyridine, collidine or quinoline.

The carbon atom marked * in formula (I) is asymmetric and the compound may be derived from the side-chain having a D, L or DL configuration at that position. All forms of compound (I) are included in

this invention. Suitably, the carbon atom marked * is derived from the D-configuration and is conveniently referred to as the D-penicillin derivative.

Compounds within formula (I) may occur in two or more tautomeric forms, all such forms are within the scope of the present invention.

In formula (I), the group R is preferably 2- or 3-thienyl, 2- or 3-furyl, 2-aminothiazolyl, cyclohexyl, cyclohexen-1-yl, cyclohexa-1,4-dien-1-yl, or mono- or di-substituted phenyl wherein the substituents are selected from halogen, especially fluorine and chlorine, hydroxy, acetoxy and methoxy. In particular R may represent phenyl, 4-hydroxyphenyl, 3,4-dihydroxyphenyl, 3,4-diacetoxyphenyl, or 2 or 3-thienyl,2- or 3-furyl, 2-,3, or 4 - chlorophenyl, or 2-, 3-, or 4-fluorophenyl.

Suitably $R^1$ represents a substituted amino group.

More suitably $R^1$ represents an amino group substituted by an optionally substituted phenyl group. The substituent for the phenyl group is preferably in the para position and is selected from amino, alkylamino, $C_{1-6}$ alkyl, hydroxy, alkoxy and aminosulphonyl.

Preferred groups $R^1$ are aminosulphonylphenylamino, and phenylamino

Specific compounds within this invention include the following:

6,β-[D-2-[3-[2-(4-aminosulphonylphenylamino)-4-hydroxypyrimidin-5-yl]ureido]-2-phenylacetamido]-6,α-methoxypenicillanic acid; and

6β-[D-2-[3-[4-hydroxy-2-(phenylamino)pyrimidin-5-yl]ureido]-2-phenylacetamido]-6α-methoxy-penicillanic acid; and

6,β-[D-2-[3-[2-(4-aminosulphonylphenylamino)-4-hydroxypyrimidin-5-yl]ureido]-2-(4-hydroxyphenyl)acetamido]-6,α-methoxypenicillanic acid; and

6β-[D-2-[3-[2-(4-aminosulphonylphenylamino)-4-hydroxy pyrimidin-5-yl]ureido]-2-(thien-2-yl)acetamido]-6,α-methoxypenicillanic acid; and

6,β-[L-2-[3-[2-(4-aminosulphonylphenylamino)-4-hydroxy-pyrimidin-5-yl]ureido]-2-thien-2-yl)acetamido]-6α-methoxypenicillanic acid; and

6,β-[D,L-2-[3-[2-(4-aminosulphonylphenylamino)-4-hydroxypyrimidin-5-yl]-ureido]-2-(furan-2-yl) acetamido]-6,α-methoxy penicillanic acid; and

6,β-[D,L-2-[3-[2-(4-aminosulphonylphenylamino)-4-hydroxypyrimidin-5-yl]ureido]-2-(2-fluorophenyl) acetamido]-6,α-methoxypenicillanic acid; and

6,β-[D,L-2-[3-[2-(4-aminosulphonylphenylamino)-4-hydroxy-pyrimidin-5-yl]ureido]-2-(4-fluorophenyl)-acetamido]-6,α-methoxypenicillanic acid; and

6,β-[D,L-2-[3-[2-(4-aminosulphonylphenylamino)-4-hydroxypyrimidin-5-yl]ureido]-2-(3,4-diacetoxyphenyl) acetamido]-6,α-methoxypenicillanic acid.

The compounds of formula (I) may be prepared by reacting a compound of formula (II):

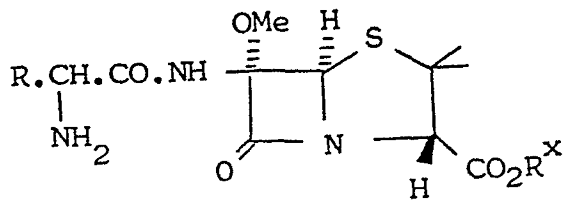

(II)

wherein the amino group is optionally substituted with a group which permits acylation to take place, R is as defined with respect to formula (I) and any reactive substituents may be protected, and $R^x$ is hydrogen or a carboxyl-blocking group, with an N-acylating derivative of an acid of formula (III):

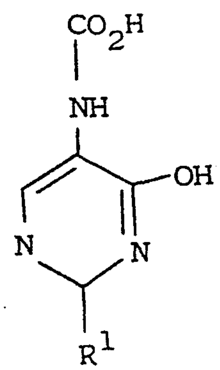

(III)

wherein $R^1$ is as defined with respect to formula (I) above, and any reactive groups may be protected; and thereafter, if necessary, carrying out one or more of the following steps:

i) removing any carboxyl-blocking group $R^x$;
ii) removing any protecting groups on the side-chain group;
iii) converting the product into a salt or in vivo hydrolysable ester thereof.

Suitable groups which permit acylation to take place and which are optionally present on the amino group of the starting material of the formula (II)

include N-silyl, N-stannyl and N-phosphorus groups, for example trialkylsilyl groups such as trimethylsilyl, trialkyltin groups such as tri-n-butyltin, groups of formula $-P.R^aR^b$ wherein $R^a$ is an alkyl, haloalkyl, aryl, aralkyl, alkoxy, haloalkyl, aryl, aralkyl, alkoxy, haloalkoxy, aryloxy, aralkyloxy or dialkylamino group, $R^b$ is the same as $R^a$ or is halogen or $R^a$ and $R^b$ together form a ring; suitable such phosphorus groups being $-P(OC_2H_5)_2$, $-P(C_2H_5)_2$,

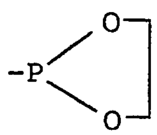 and 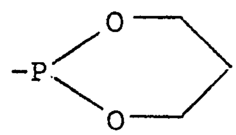

Suitable carboxyl-blocking derivatives for the group $-CO_2R^x$ in formula (II) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with trilower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl,

methoxymethyl, a silyl, stannyl or phosphorus-containing group, such as described above, an oxime radical of formula $-N=CHR^o$ where $R^o$ is aryl or heterocyclic, or an *in vivo* hydrolysable ester radical such as defined above.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

A reactive N-acylating derivative of the acid (III) is employed in the above process. The choice of reactive derivative will of course be influenced by the chemical nature of the substituents of the acid.

Suitable N-acylating derivatives include an acid halide, preferably the acid chloride or bromide. Acylation with an acid halide may be affected in the presence of an acid binding agent for example, tertiary amine (such as triethylamine or dimethylaniline), an inorganic base (such as calcium carbonate or sodium bicarbonate) or an oxirane, which binds hydrogen halide liberated in the acylation reaction. The oxirane is preferably a $(C_{1-6})$-1,2,alkylene oxide - such as ethylene oxide or propylene oxide. The acylation reaction using an acid halide may be carried out at a temperature in the range -50°C to +50°C, preferably -20°C to +20°C, in aqueous or non-aqueous media such as aqueous acetone, aqueous tetrahydrofuran, ethyl, acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The intermediate compound of formula (II) may be prepared by reacting a compound of formula (IV):

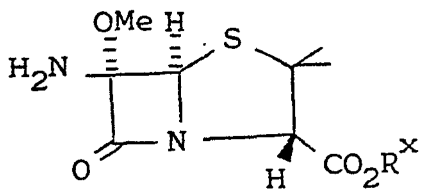

(IV)

wherein the amino group is optionally substituted with a group which permits acylation to take place and $R^x$ is as defined with respect to formula (II) above, with an N-acylating derivative of an acid of formula (V):

$$\underset{\displaystyle NHR^z}{R.\underset{|}{C}H.CO_2H} \qquad (V)$$

wherein R is as defined with respect to formula (I) and any reactive groups therein may be protected and $R^z$ is an amino-protecting group; and thereafter removing protection group $R^z$.

Suitable N-acylating derivatives, carboxyl protecting groups and reaction conditions include those described hereinbefore.

Suitable amino-protecting groups $R^z$ are those well-known in the art which may be removed under conventional conditions without disruption of the remainder of the molecule.

The starting material of formula (IV) is disclosed in British Patent No. 1 339 007.

The compound of formula (I) may also be prepared by reacting a compound of formula (IV) as described hereinbefore with an N-acylating derivative of an acid of formula (VI):

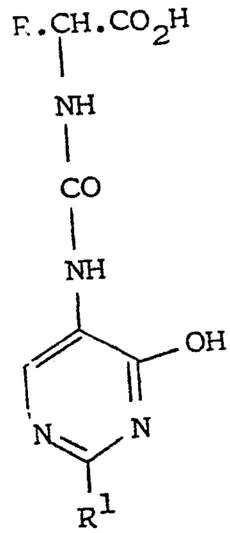

(VI)

wherein R and $R^1$ are as defined with respect to formula (I) and any reactive groups therein may be protected; and thereafter, if necessary, carrying out one or more of the following steps:

i) removing any carboxyl-blocking group $R^x$;
ii) removing any protecting groups on the side-chain group;
iii) converting the product into a salt or <u>in vivo</u> hydrolysable ester thereof.

Compounds of formula (I) may also be prepared by reacting a compound of formula (VII):

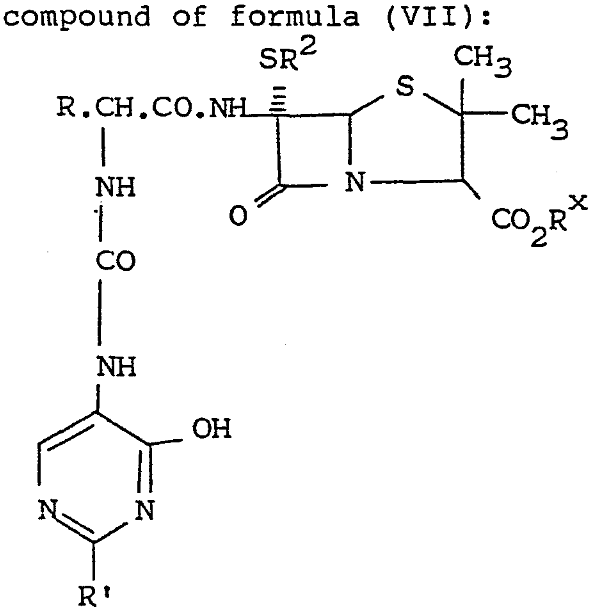

(VII)

wherein R, $R^1$ and $R^x$ are as defined above and $R^2$ is $C_{1-6}$ alkyl, aryl or benzyl;

(A) with methanol in the presence of a metal ion, such as mercury, lead, silver, cadmium, thallium, or bismuth; or

(B) with chlorine or bromine at -25°C to -80°C and subsequently decomposing the resultant halosulphonium halide with methanol and a base.

The intermediate compound of formula (II) may also be prepared by reacting a compound of formula (VIII):

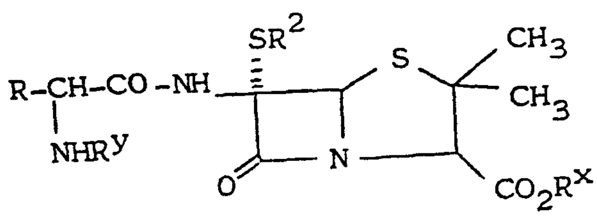

(VIII)

wherein R, $R^2$ and $R^x$ are as defined above and $R^y$ is hydrogen or a group $R^z$ as defined above;

(A) with methanol in the presence of a metal ion, such as mercury, lead, silver, cadmium, thallium or bismuth; or

(B) with chlorine or bromine at -25°C to -80°C and subsequently decomposing the resultant halosulphonium halide with methanol and a base.

The reaction with methanol in the presence of a metal ion may generally be carried out at a temperature of from -50°C to +25°C, but is conveniently carried out between -5°C and +25°C. Any suitable solvent may be employed. It is generally convenient however to use methanol as the solvent. Preferably $R^2$ represents methyl.

Compounds of formula (I) may also be prepared by:

a) treating a compound of formula (IX)

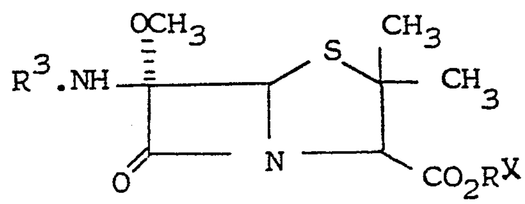

(IX)

wherein $R^x$ is a carboxyl-blocking group, and $R^3$ is an acyl group, in particular an acyl group derived from side-chain of a natural penicillin, such as benzyl penicillin or phenoxymethyl penicillin; with an agent forming an imino halide;

b) treating the imino halide with a compound to introduce a group $QR_f$ on the imino carbon atom, wherein Q is oxygen, sulphur or nitrogen and $R_f$ is an alkyl group of from 5 to 14 carbon atoms, to form an iminoether, iminothioether, or amidine (when Q is O, S, or N respectively);

c) reacting with an N-acylating derivative of an acid of formula (VI);

d) treating with water; and

e) optionally removing the carboxyl-blocking group $R^x$.

A suitable agent for preparing an imino halide is an acid halide in the presence of an acid binding agent such as a tertiary amine, eg. pyridine, triethylamine, or N,N-dimethylaniline. Examples of suitable acid halides are phosphorus pentachloride, phosgene, phosphorous pentabromide, phosphorus oxychloride, oxalyl chloride and p-toluene sulphonic acid chloride.

Phosphorus pentachloride and phosphorus oxychloride are preferred. The reaction may be conducted under cooling, preferably at temperatures from $0^{o}C$ to $-30^{o}C$ when phosphorus pentachloride is employed. The amount of the tertiary amine is preferably 3 - 5 mols per mol of phosphorus pentachloride. It is also preferable to use the phosphorus halide in an amount slightly in excess of that of the starting material.

The resulting imino compounds are then treated to introduce a $-QR_f$ group onto the imino carbon atom. This is preferably effected by reacting the imino halide with a corresponding alcohol. Examples of suitable alcohols for reaction with the imino halide are aliphatic alcohols containing from 1 to 12 carbon atoms, preferably 1 to 5 carbon atoms, such as methanol, ethanol, propanol, isopropyl alcohol, amyl alcohol and butyl alcohol, and aralkyl alcohols such as benzyl alcohol and 2-phenylethanol.

The reaction of the alcohol with the imino halide is preferably effected in the presence of an acid binding agent, such as a tetiary amine, preferably pyridine, and the reaction is usually carried out without isolating the imino halide from the reaction mixture.

Finally, the product is treated with water. The water treatment may be conducted together with the isolation of the desired material. That is the reaction mixture may be added to water or a saturated aqueous solution of sodium chloride and then the aqueous layer formed is separated from the organic solvent layer.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The composition may be formulated for administration by any route, such as oral topical or parenteral. The compositions may be in the form of tablets, capsules, powders, granules, lozenges, creams or liquid preparations, such as oral or sterile parenteral solutions or suspensions.

Tablets and capsules for administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin,

sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parental suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration.

The compound of formula (I) may be the sole therapeutic agent in the compositions of the invention or a combination with other antibiotics or with a β-lactamase inhibitor may be employed.

Advantageously, the compositions also comprise a compound of formula (X) or a pharmaceutically acceptable salt or ester thereof:

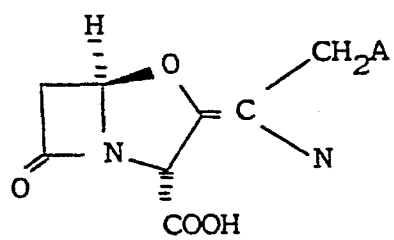

(X)

wherein A is hydroxyl, substituted hydroxyl, thiol, substituted thiol, amino, mono- or di-hydrocarbyl-substituted amino, or mono- or di-acylamino.

A further advantageous composition comprises a compound of formula (I) or a pharamceutically acceptable salt or in vivo hydrolysable ester thereof together with a compound of formula (XI) or a pharmaceutically acceptable salt or in vivo hydrolysable ester thereof:

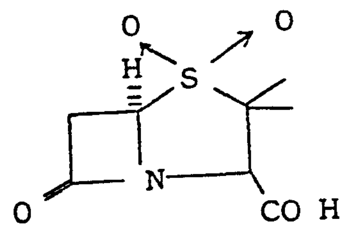

(XI)

The present invention also provides a method of treating bacterial infections in animals, in particular humans or domestic mammals, which comprises the administration of the composition of this invention

The following Examples illustrate the preparation of the compounds of this invention.

Example 1

Sodium 6,β-[D-2-[3-[2-(4-aminosulphonylphenylamino)-4-hydroxypyrimidin-5-yl]ureido]-2-phenylacetamido] -6,α-methoxypenicillanate

A suspension of 2-(4-aminosulphonylphenylamino)-5-amino-4-hydroxypyrimidine (0.28g, 1 mmol) in freshly distilled, THF (50 ml) with triethylamine at 0°C was treated with phosgene (1 ml of 12.5% w/w solution in toluene, 1.1 mmol). After 15 minutes the reaction was allowed to gain room temperature, stirred for 30 minutes, evaporated to half volume and added dropwise to a solution of 6,β-(D-2-amino-2-phenylacetamido)-6,α-methoxypenicillanic acid (0.433g, 1 mmol) in 60% aqueous-THF (50 ml) at pH 7.5 with triethylamine. The pH was maintained between 7.5 and 8.0 by additions of triethylamine over 1½ hours. The THF was evaporated in vacuo and the aqueous residue washed with ether (2 x 50 ml), layered with ethylacetate (20 ml) and the pH adjusted to 2.0 with 5N hydrochloric acid. The precipitated solid and the aqueous phase were discarded. The ethyl acetate phase was washed with water (3 x 20 ml), dried over magnesium sulphate, filtered and evaporated to dryness in vacuo. The gum was redissolved in acetone (20 ml) and 2N sodium 2-ethylhexanoate in 4-methylpentan-2-one added until precipitation was complete. The solid was filtered off washed thoroughly with ether and dried in vacuo to yield 0.14 g, 19.8%. $\nu_{max}$ (KBr) 3320 (b), 1760, 1655, 1580, 1530, 1340, 1160 and 1100 $cm^{-1}$, δ [$(CD_3)_2SO$,$CD_3OD$] 1.02, 1.30 (6H, 2 x s, 2 x $2CH_3$), 3.45 (3H, s, $OCH_3$), 4.06 (1H, s, 3H), 5.42, 5.47 (2H, 2 x s, 5H, CHCON), 7.15-8.05 (9H, m, Ph, Ar,), 8.26(1H,s, pyrimidin 3H). Antibacterial activity (MIC µg/ml): E Coli JT 425, 2.5; Ps aeruginosa 10662, 50; Klebsiella aerogenes A, 2.5; P mirabilis C977, 2.5; Staph. aureus Oxford, 10.

Example 2

Sodium 6β-[D-2-[3-[4-hydroxy-2-(phenylamino)pyrimidin-5-yl]ureido]-2-phenylacetamido]-6α-methoxy-penicillanate

6β-[D-2-Amino-2-phenylacetamido]-6α-methoxy-penicillanic acid trihydrate (0.39 g, 0.9 mmol) was suspended in water (10 ml) and tetrahydrofuran (20 ml).

The pH of the mixture was adjusted to 7.5 with triethylamine and the resulting solution treated portionwise with N-[4-hydroxy-2-(phenylamino)pyrimidin-5-yl]carbamoyl chloride (0.24 g, 0.9 mmol), the pH of the mixture being maintained between 7 and 7.5 by addition of triethylamine. After 0.5 h at room temperature, the tetrahydrofuran was evaporated *in vacuo*, the aqueous residue washed with ethyl acetate (30 ml), diethyl ether (30 ml) and covered with a layer of ethyl acetate (30 ml). The pH of the aqueous phase was adjusted to 2 by addition of 5M hydrochloric acid, the phases separated, the aqueous phase further extracted with ethyl acetate (2 x 20 ml), the extracts combined, washed with water at pH2 (20 ml), water (20 ml) and saturated brine (20 ml), before drying over anhydrous magnesium sulphate. Evaporation of the solvent *in vacuo*, gave a yellow powder (0.34 g, 62%), which was dissolved in acetone (20 ml) and methanol (2 ml) and the resulting solution treated with 2M sodium 2-ethylhexanoate in 4-methylpentan-2-one (0.28 ml, 0.56 mmol) followed by diethyl ether (100 ml). The precipitate was collected by filtration, washed with ether and dried *in vacuo* to yield the title compound, 0.305g, 53%, $\nu_{max}$ (KBr) 3330b, 1760, 1657b, 1600b, 1540b, 1498, 1443, 1390, 1372, 1340, 1255sh, 1211 and 1100 $cm^{-1}$; δ [$(CD_3)_2SO$] 1.00 and 1.29 (6H, 2s, $2x2CH_3$), 3.40 (3H, s, $OCH_3$), 3.91 (1H, s, 3-H), 5.35 (1H, s,

5-H), 5.52 (1H, d J 8Hz, CH), 6.76-7.92 (12H, m, 2xPh and 2 x NH), 8.18 (2H, bs, pyrimidine proton and NH), 9.57 (1H, bs, CONH), 10.90 (1H, bs, OH). Antibacterial activity (MIC µg/ml):E Coli JT 425, 12.5; Klebsiella aerogenes A, 5; P mirabilis C977, 12.5; Staph. aureus Oxford, 12.5.

Example 3

Sodium 6,β-[D-2-[3-[2-(4-aminosulphonylphenylamino)-4-hydroxypyrimidin-5-yl]ureido]-2-(4-hydroxyphenyl)acetamido]-6,α-methoxypenicillanate

i) Benzyl 6β-[D-2-(4-benzyloxycarbonyloxyphenyl)-2-(4-nitrobenzyloxycarbonylamino)acetamido]-6α-methylthio penicillanate.

D-2-(4-Benzyloxycarbonyloxyphenyl)-2-(4-nitrobenzyloxy carbonylamino) acetic acid (4.80g;10mmol) in dry dichloromethane (50ml) was treated with oxalyl chloride (1.0ml) and dry DMF (5 drops) at room temperature. After 1 hour the solvent was removed in vacuo and the residue redissolved in dry THF (20ml). This solution was added dropwise, at 0-5$^0$C, to a solution of benzyl 6β-amino-6α-methylthiopenicillanate (3.52g; 10mmol) in THF (20ml) containing pyridine (0.8ml;10mmol). Stirred at 0-5$^o$C for 0.5 hours and allowed to regain room temperature over a further 0.5 hours. The reaction mixture was then evaporated to dryness in vacuo and the residue treated with ethyl acetate (100ml) and water (100ml). The phases were separated and the organic phase washed with water (50ml), saturated brine (25ml), dried over anhydrous magnesium sulphate and evaporated to dryness in vacuo to yield a yellow foam. Crystallisation from ethyl acetate-cyclohexane gave the title compound (4.14g; 51% yield) as a white,crystalline solid, m.p 103-105$^o$C, $[\alpha]_D^{20}$ + 46.3$^0$

(C=1 in $CHCl_3$), $\nu_{max}$(KBr) 3000b, 1775, 1760, 1740, 1670, 1515, 1348, 1240b, 1053 and 700cm$^{-1}$, δ($CDCl_3$), 0.95 and 1.21 (6H, 2xs, 2x2$CH_3$), 2.25(3H,s, $SCH_3$), 4.33 (1H,s,3-H), 5.17 and 5.25(6H,2xs, 2x2$\underline{CH_2}$Ph and

0091755

$\underline{CH_2}C_6H_4$), 5.45-5.70 (2H,m,5-H and CH), 6.41(1H,dJ7Hz $\underline{NH}$CH), 6.98- 7.64 and 8.10-8.35(19H,m, 4xAr and CONH). Found: C,59.03, H, 5.12; N, 6.52; S,7.69%. $C_{40}H_{38}N_4O_{11}S_2$ requires; C,58.97; H, 4.67; N, 6.88; S, 7.86%.

ii) Benzyl6β-[D-2-(4-benzyloxycarbonyloxyphenyl)-2-(4-nitrobenzyloxycarbonylamino)acetamido]-6α-methoxypenicillanate.

Benzyl 6β-[D-2-(4-benzyloxycarbonyloxyphenyl)-2-(4-nitrobenzyloxycarbonylamino) acetamido]-6α-methylthiopenicillanate (1.58g; 1.9mmol) was suspended in methanol (150ml). Just sufficient DMF was added to obtain complete solution when mercuric acetate (0.61g; 1.9mmol) was added. After 0.5hours at room temperature, the mixture was filtered and the filtrate evaporated to dryness *in vacuo* to yield an off-white foam, which was treated with ethyl acetate (150ml) and water (50ml). The phases were separated and the organic phase washed with water (3x50ml), brine (50ml), dried over anhydrous magnesium sulphate and evaporated to dryness *in vacuo* to yield a white powder. Recrystallisation from ethyl acetate-cyclohexane gave the product (1.39g;90%), m.p 107-109$^0$C, $\nu_{max}$(KBr) 3410, 3310, 1765, 1690, 1520, 1500, 1350, 1240, 1100, and 1050cm$^{-1}$, δ($CDCl_3$)0.87 and 1.18 (6H, 2xs, 2x2-$CH_3$), 3.43(3H,s,$OCH_3$), (4.28(1H,s,3-H), 5.13 and 5.21 (6H, 2xs, 2x$\underline{CH_2}C_6H_5$ and $\underline{CH_2}C_6H_4$), 5.45(1H,d,J8Hz,CH), 5.55(1H,s,5-H), 6.30(1H,d J8Hz, $\underline{NH}$CH), 7.00-7.57 and 8.09-8.26(19H,m,aromatics and CONH). Found: C, 58.74; H, 4.98; N, 6.85; S, 4.41%. $C_{40}H_{38}N_4O_{12}S$ requires: C,60.15; H, 4.76; N,7.02; S, 4.01%.

iii) 6β-[D-2-Amino-2-(4-hydroxyphenyl)acetamido]-6α-methoxypenicillanic acid.

Benzyl 6β-[D-2-(4-benzyloxycarbonyloxyphenyl)-2-(4-nitrobenzyloxycarbonylamino)acetamido]-6α-methoxy penicillanate (1.3g; 1.63mmol) was dissolved in THF (25ml) and the soluton diluted with ethanol (100ml). Hydrogenation at atmospheric pressure and room temperature was accomplished over 10% palladium on charcoal in 1 hour. The catalyst was removed by filtration and the filtrate concentrated to low volume in vacuo and the residue diluted with ether (100ml). The resulting solid was filtered, washed with ether and dried in vacuo 0.49g; 76%, $\nu_{max}$ (KBr) 3700-2200b, 1760, 1680, 1600b, 1515, 1255b, and 1100cm$^{-1}$, $\delta$[$(CD_3)_2SO$]0.95 and 1.28 (6H,2xs, 2x2-$CH_3$), 3.39 (3H,s,$OCH_3$), 4.02(1H,s,3-H), 4.81(1H,bs,CH), 5.32(1H,s,5-H), 6.75 and 7.34(4H,2d J9Hz, $C_6H_4$).

iv) Sodium 6,β-[D-2-[3-[2-(4-aminosulphonyl phenylamino)-4-hydroxypyrimidin-5-yl]ureido] -2-(4-hydroxyphenyl)acetamido]-6,α-methoxypenicillanate

A suspension of 2-(4-aminosulphonylphenylamino)-5-amino-4-hydroxypyrimidine (0.28g, 1mmol) in freshly distilled THF (50 ml) with triethylamine (0.14 ml, 1 mmol) at 0°C was treated with phosgene (1 ml of 12.5% w/w solution in toluene, 1.1 mmol). After 15 minutes the reaction was allowed to gain room temperature, stirred for 30 minutes and added dropwise to a solution of 6,β-[D-2-amino-2-(4-hydroxyphenyl)acetamido]-6,α -methoxypenicillanate (0.419g, 1 mmol) in 60% aqueous THF (50 ml) at pH 7.5 with triethylamine. The pH was maintained between 7.5 and 8.0 by additions of triethylamine over 1.5 hours. The THF was evaporated in vacuo and the aqueous residue washed with ether (2x50 ml), layered with ethyl acetate (20 ml) and the

pH adjusted to 2.0 with 5N hydrochloric acid. The precipitated solid and the aqueous phase were discarded. The ethyl acetate phase was washed with water (3x20 ml), dried over magnesium sulphate, filtered and evaporated to dryness in vacuo. The gum was redissolved in acetone (10 ml) and 1.85 N sodium 2-ethylhexanoate in 4-methylpentan-2-one added until precipitation was complete. The solid was filtered off, washed thoroughly with acetone, ether and dried in vacuo and chromatographed on silica gel eluting with ethyl acetate, ethanol, water (6:2:1) to yield 16 mg. $\nu_{max}$ (KBr) 3450 (b), 1760, 1655, 1590, 1530, 1340 and 1155 $cm^{-1}$; $\delta$ [$(CD_3)_2SO$, $CD_3OD$] 1.00, 1.28 (6H, 2xs, $2xCH_3$), 3.41 (3H, s, $OCH_3$), 3.97 (1H, s, 3H) 5.31 (1H, s, CHCON), 5.35 (1H, s, 5H), 6.70, 7.28 (4H, ABq, J10Hz, HO-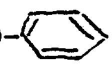-), 7.72, 7.76 (4H, ABq, J10Hz, -N-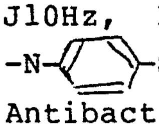-$SO_2$-), 8.24 (1H, s, pyrimidin 3H). Antibacterial activity (MIC µg/ml): E Coli JT 425, 2.5; Ps aerugimosa 10662, 25; Klebsiella aerogenes A, 0.2; P mirabilis C977, 1.25; Staph, aureus Oxford, 50.

Example 4

Sodium 6β-[D-2-[3-[2-(4-aminosulphonylphenylamino)-4-hydroxy pyrimidin-5-yl]ureido]-2-(thien-2-yl)acetamido]-6,α-methoxypenicillanate.

i) Benzyl 6α-methylthio-6β-[DL-2-(4-nitrobenzyloxy carbonylamino)-2-(thien-2-yl)acetamido]penicillanate.

DL-2-(4-Nitrobenzyloxycarbonylamino)-2-(thien-2-yl)acetic acid (3.36g; 10mmol) was suspended in dry dichloromethane (25ml) and treated at room temperature with oxalyl chloride (1.0ml) and dry DMF (3 drops). After 1 hour at room temperature, the reaction mixture was evaporated to dryness in vacuo and the residue redissolved in dry THF (15ml). Benzyl 6β-amino-6α-methylthiopenicillanate (3.52g; 10mmol) was dissolved in THF (15ml) containing triethylamine (1.4ml; 10mmol) and the mixture treated at room temperature with the acid chloride solution. After 1 hour at room temperature, the reaction was filtered. The filtrate was evaporated to dryness in vacuo and the resulting yellow foam chromatographed on silica gel eluting with 10% ethyl acetate in cyclohexane grading to 20% ethyl acetate in cyclohexane to yield (5.18g; 77%) as a foam, $\nu_{max}$ (KBr) 3400, 3300b, 1780, 1740, 1680, 1520, 1347, 1320, 1310, 1230b, 1205, 1055 and 700cm$^{-1}$, δ($CDCl_3$) 1.10, 1.24, 1.30 and 1.35(6H, 4xs, 2x2-$CH_3$), 2.02 and 2.20(3H, 2xs, $SCH_3$), 4.35 and 4.40 (1H, 2xs, 3-H), 2.10-2.27(4H,m,$\underline{CH_2}C_6H_5$ and $\underline{CH_2}C_6H_4$), 5.53 (1H,s,5-H), 5.70-5.95(1H,m,NH$\underline{CH}$), 6.31 (1H,dJ8Hz, $\underline{NH}$CH), 6.81-7.60 and 8.03-8.28(12H,2m, thienyl, $C_6H_4$ and $C_6H_5$), 7.75(1H,b, CONH). Found: $\underline{M}^+$ 670.1194. $C_{30}H_{30}N_4O_8S_3$ requires 670.1225.

ii) Benzyl 6α-methoxy-6β-[D and L-2-(4-nitrobenzyloxy carbonylamino)-2-(thien-2-yl)acetamido]penicillanate

Benzyl 6α-methylthio-6β-[DL-2-(4-nitrobenzyloxycarbonylamino)-2-(thien-2-yl)acetamido]penicillanate (3.52 g; 5.25 m mol) in methanol (100 ml), was treated at room temperature with mercuric acetate (1.67 g; 5.25 m mol). After 0.5 hours, the reaction mixture was diluted with ethyl acetate (200 ml), filtered and washed with water (100 ml), saturated brine (100 ml), dried over anhydrous magnesium sulphate and evaporated to dryness *in vacuo*. Preparative high performance liquid chromatography (Waters prep 500 machine, Waters prep-pak 500/silica column and 30% ethyl acetate in cyclohexane as eluent) gave the D-diasteroisomer (1.09 g; 32%) as a foam, $[\alpha]_D^{20}$ + 100.3°(C=1 in chloroform), $\nu_{max}$ (KBr) 3310 b, 1775, 1742, 1685, 1520, 1348, 1320, 1260, 1240, 1206, 1100, 1055 and 700 $cm^{-1}$, δ ($CDCl_3$) 1.08, 1.25 (6H, 2xs, 2x2-$CH_3$), 3.42 (3H, s, $OCH_3$), 4.35 (1H, s, 3-H), 4.15 and 4.22 (4H, 2xs, $\underline{CH_2}C_6H_5$ and $\underline{CH_2}C_6H_4$), 5.59 (1H, s, 5-H), 5.83 (1H, d, 3 8 Hz, C$\underline{H}$NH), 6.29 (1H, d J8Hz, CHN$\underline{H}$), 6.80-7.00, 7.13-7.58, 8.00 -8.25 (12H, 3m, $C_6H_5$, $C_6H_4$ and thienyl protons), 7.60-8.00 (1H, bs, CONH). Found: $\underline{M}^+$ 654.1405. $C_{30}H_{30}N_4O_9S_2$ requires 654.1454.

Also obtained was the L diastereoisomer (0.97 g; 28%, $[\alpha]_D^{20}$ + 171.8° (C=1 in chloroform), $\nu_{max}$ (KBr) 3400b, 3320b, 1780, 1740, 1690, 1520, 1347, 1205, 1102, and 700 $cm^{-1}$; δ($CDCl_3$) 1.31 and 1.36 (6H, 2xs, 2x2-$CH_3$), 3.28 (3H, s, $OCH_3$), 4.40 (1H, s, 3-H), 5.17 (4H, s, C$\underline{H}_2C_6H_5$ and C$\underline{H}_2C_6H_4$), 5.53 (1H, s, 5-H), 5.59 (1H, d J 7Hz, CH), 6.13 (1H, d J 7Hz, N$\underline{H}$CH), 6.88-7.60 and 8.05-8.28 (13H, m, $C_6H_4$, $C_6H_5$, thienyls, CONH). Found: $\underline{M}^+$ 654.1399. $C_{30}H_{30}N_4O_9S_2$ requires $\underline{M}$ 654.1454.

iii) 6,β-(D-2-Amino-2-(thien-2-yl)acetamido)-6α-methoxypenicillanic acid

Benzyl 6α-methoxy-6β-[D-2-(4-nitrobenzyloxycarbonyl-amino)-2-thien-2-yl) acetamido]penicillanate (1.0 g, 1.5 m mol) was dissolved in THF (minimum), the solution diluted to turbidity with water then diluted to a clear solution by addition of ethanol. Hydrogenation over 10% palladium on charcoal at room temperature and atmospheric pressure for 2 hours, followed by filtration and evaporation to dryness *in vacuo* gave a crude product, which was triturated under a mixture of propan-2-ol (5 ml) and diethyl ether (20 ml) and the resulting solid collected by filtration, washed with ether and dried *in vacuo* (0.27 g; 46%), $\nu_{max}$ (KBr) 3700-2200 b, 1760, 1700, 1605, 1525b, 1400, 1370, 1345, 1250 and 1095cm$^{-1}$, δ[$(CD_3)_2SO$] 1.15 and 1.31 (6H, 2xs, 2x2-$CH_3$), 3.37 (3H, s, $OCH_3$), 4.10 (1H, s, 3-H), 4.97 (1H, s, CH), 5.32 (1H, s, 5-H), 6.90-7.05, 7.15-7.28, 7.35-7.50 (3H, 3m, thienylprotons).

iv) Sodium 6,β-[D-2-[3-[2-(4-aminosulphonylphenyl amino)-4-hydroxypyrimidin-5-yl]-ureido]-2-(thien-2-yl) acetamido]-6α-methoxypenicillanate.

Prepared by the method described in example 1 except that 6,β-[D-2-amino-2-(thien-2-yl)acetamido]-6α-methoxypenicillanic acid (0.385 g, 1 m mol) was used. The product was chromatographed on silica gel eluting with ethyl acetate, ethanol, water (6:2:1) to yield 70 mg, $\nu_{max}$ (KBr) 3320 (b), 1765, 1655, 1590, 1535 (b) and 1155 cm$^{-1}$, δ$(CD_3)_2SO,CD_3OD$) 1.19, 1.37 (6H, 2xs, 2x2 $CH_3$), 3.43 (3H, s, $OCH_3$), 4.06 (1H, s, 3H), 5.47 (1H, s, 5H), 5.78 (1H, s, CHCON), 6.89-7.50 (3H, m, thienyl 3,4 and 5H) 7.79
(4H, s,N-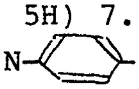-$SO_2$-), 8.30 (1H, s, pyrimidin 3H).

Example 5

Sodium 6,β-[L-2-[3-[2-(4 aminosulphonylphenylamino)-4-hydroxy-pyrimidin-5-yl]ureido]-2-thien-2-y-)acetamido]-6α-methoxypenicillanate

i) 6β-(L-2-Amino-2-(thien-2-yl)acetamido)-6α-methoxy-penicillanic acid

Benzyl 6α-methoxy-6β-[L-2-(4-nitrobenzyloxycarbonyl-amino)-2-(thien-2-yl)-acetamido]penicillanate (0.43 g; 0.66 m mol) from example 4ii was dissolved in minimum THF, the solution diluted to turbidity with water then diluted with ethanol to give a clear solution. Hydrogenation was carried out over 10% palladium on charcoal at room temperature and atmospheric pressure. After 4 hours, the catalyst was removed by filtration, the filtrate concentrated in vacuo, washed with chloroform, filtered through Celite and evaporated to dryness in vacuo (0.24 g, 96%), $\nu_{max}$ (KBr) 3800-2400, 1768, 1670 (b), 1600 (b), 1510, 1250, 1170 and 1110 $cm^{-1}$, $\delta[(CD_3)_2SO]$ 1.42 (6H, s, 2x2-$CH_3$), 3.22 (3H, s, $OCH_3$), 4.16 (1H, s, 3-H), 4.85 (1H, s, CH), 5.38 (1H, s, 5-H), 6.90-7.05, 7.08-7.20 and 7.32-7.50(3H, 3m, thienyl protons.

ii) Sodium 6,β-[L-2-[3-[2-(4-aminosulphonylphenylamino)-4-hydroxy-pyrimidin-5-yl]ureido]-2-(thien-2-yl)acetamido]-6,α-methoxypenicillanate

Prepared by the method described in example 1 except 6,β-[L-2-amino-2-(thien-2-yl)acetamido]-6,α-methoxy-penicillanic acid (0.385 g, 1mmol) was used. The product was chromatographed on silica gel eluting with ethyl acetate, ethanol, water (6:2:1) to yield 110 mg $\nu_{max}$ (KBr) 3350 (b), 1765, 1660, 1590, 1530 (b) and

1155 $cm^{-1}$, δ[$(CD_3)_2SO+CD_3OD$] 1.47 (6H, s, $2x2CH_3$), 3.26 (3H,s,$OCH_3$), 4.13 (1H, s, 3H), 4.48 (1H, s, 5H), 5.87 (1H, s, CHCON), 6.90-7.45 (3H, m, thienyl 3,4 and 5H),

7.78, 7.84 (4H, ABq J10Hz, -N- 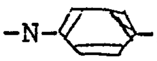 $-SO_2$), 8.30 (1H, s, pyrimidin 3H)

Example 6

Sodium 6,β-[D,L-2-[3-[2-(4-aminosulphonylphenylamino)-4-hydroxypyrimidin-5-yl]-ureido]-2-(furan-2-yl) acetamido]-6,α-methoxy penicillanate

i) Benzyl 6,β-[D,L-2-benzyloxycarbonylamino-2-(fur-2-yl)acetamido]-6,α-methylthiopenicillanate

2-Benzyloxycarbonylamino-2-(fur-2-yl)acetic acid (1.38 g, 5 mmol) in dichloromethane (15 ml) was added dropwise over 30 minutes to an ice bath cooled solution of benzyl 6,β-amino-6,α-methylthiopenicillanate (1.94 g 5.5 mmol) and DCC (1.13g 5.5 mmol) in dichloromethane (30 ml). The mixture was stirred overnight then filtered and the filtrate evaporated to dryness. The residue was chromatographed on silica gel eluting with 20% ethyl acetate in cyclohexane to give the title compound, 2.37 g 77.8% yield, δ($CDCl_3$) 1.20, 1.25, 1.32, 1.42 (6H, 4xs, 2x2 $CH_3$), 2.08, 2.22(3H, 2xs, $SCH_3$), 4,36, 4.41 (1H, 2xs, 3H), 5.08, 5.12 (4H, 2xs, 2xO$\underline{CH_2}$Ph), 5.50 (1H, s, 5H), 5.5-5.9 (1H, m, $\underline{C}$HCONH), 6.0-6.4 (3H, m, furan 3H and 4H, NHCO), 7.1-7.4 (11H, m, 2xPh, furan 5H), 7.90-8.05 (1H, 2xs, CONH).

ii) Benzyl 6,β-[D,L-2-benzyloxycarbonylamino-2-(fur-2-yl)acetamido]-6α-methoxypenicillanate

Mercuric acetate (1.24 g 3.89 mmol) was added to benzyl 6,β-[D,L-2-benzyloxycarbonylamino-2-(fur-2-yl)acetamido]-6,α-methylthiopenicillanate (2.37 g, 3.89 mmol) in methanol (20 ml). After 20 minutes ethyl acetate (150 ml) was added and the solution washed with water (3 x 50 ml) and brine (25 ml), dried and evaporated to a foam which was chromatographed on silica gel eluting with 20% ethyl acetate in cyclohexane to give the title compound, 1.87 g, 83.2% yield δ($CDCl_3$) 1.17, 1.25, 1.30, 1.37 (6H, 4xs, 2x2$CH_3$), 3.30, 3.43 (3H, 2xs,

$OCH_3$), 4.40, 4.43 (1H, 2xs, 3H), 5.13, 5.15 (4H, 2xs, 2x$OCH_2$Ph), 5.60 (1H, s, 5H) 5.5-5.9 (1H, m, $CH_2$CONH), 6.2-6.5 (3H, m, furan 3H and 4H, NHCO), 7.1-7.5(11H, m, 2xPh, furan 5H), 8.00, 8.28 (1H, 2xs, CONH).

iii) 6,β-[D,L-2-Amino-2-(fur-2-yl)acetamido]-6,α-methoxypenicillanic acid

Benzyl 6,β-[D,L-2-benzyloxycarbonylamino-2-(fur-2-yl) acetamido]-6,αmethoxypenicillanate (1.86 g) in ethanol (25 ml) was diluted with water until almost cloudy then hydrogenated in the presence of 10% palladium on carbon (1.85 g) for 30 minutes. The solution was filtered, concentrated in vacuo and freeze dried to give the title compound, 0.90 g, 77.8% yield, $\nu_{max}$ (KBr) 1765, 1705, 1600, 1250, and 1092 $cm^{-1}$, $\delta(D_2O)$ 1.20, 1.37, 1.42, 1.49 (6H, 4xs, 2x2$CH_3$) 3.40, 3.55 (3H, 2xs, $OCH_3$), 4.25, 4.30(1H, 2xs, 3H) 5.4-5.7 (2H, m, 5H,CHCONH), 6.5-6.9 and 7.7-7.9 (3H, m, furan).

iv) Sodium 6,β-[D,L-2-[3-[4-aminosulphonylphenyl amino)-4-hydroxy-pyrimidin-5-yl]ureido]-2-(furan-2-yl) acetamido]-6,α-methoxypenicillanate

A suspension of 2-(4-aminosulphonylphenylamino)-5-amino-4-hydroxy-pyrimidine (0.84g, 3 mmol) in freshly distilled THF (70 ml) with triethylamine (0.42 ml, 3 mmol) at 0°C was treated with phosgene (3 ml of 12.5% w/w solution in toluene, 3.3 mmol). After 1 hour the reaction was evaporated to half volume and added dropwise to a solution of 6,β-[D,L-2-amino-2-(furan-2-yl)acetamido]-6,α-methoxy penicillanic acid (0.524 g, 1.42 mmol) in 70% aqueous -THF (50 ml) at pH 7.5. The pH was maintained between 7.3 and 7.8 by addition of triethylamine over 1½ hours. The mixture was washed with ether (3x50 ml), filtered, layered with ethyl acetate (70 ml) and acidified to pH 2.0 with 5N

hydrochloric acid. The organic phase was collected and the aqueous extracted with ethyl acetate (2x60 ml). The combined organic phases were washed with water (3x100 ml), dried over magnesium sulphate, filtered and evaporated to dryness in vacuo. The gum was redissolved in acetone (10 ml) and 1.85 N sodium 2-ethylhexanoate in 4-methylpentan-2-one added until precipitation was complete. The solid was filtered off, washed thoroughly with ether and dried in vacuo to yield 0.3 g, $\nu_{max}$ (KBr) 3300 (b), 1765, 1655, 1600 (b), 1530 (b), 1405, 1335 and 1155 $cm^{-1}$, $\delta$[$(CD_3)_2SO+D_2O$] 1.23, 1.37,
1.42 (6H, 3xs, $2xCH_3$), 3.27, 3.42 (3H, 2xs, $OCH_3$), 4.01, 4.04, (1H, 2xs, 3H), 5.43 (1H, s, 5H), 5.62, 5.67 (1H, 2xs, CHCON), 6.50 (2H, m, furan 3,4H), 7.61 (1H, m, furan 5H), 7.77, 7.87 (4H, ABq, J9Hz, N-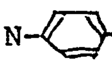-$SO_2$), 8.28. (1H, s, pyrimidin 3H).

Example 7

Sodium 6,β-[D,L-2-[3-[2-(4-aminosulphonylphenylamino)-4-hydroxypyrimidin-5-yl]ureido]-2-(2-fluorophenyl)acetamido]-6,α-methoxypenicillanate

i) Benzyl 6,β-[D,L-2-(2-fluorophenyl)-2-(4-nitrobenzyloxycarbonylamino)acetamido]-6,α-methylpenicillanate

A suspension of D,L-2-(2-fluorophenyl)-2-(4-nitrobenzyloxycarbonylamino)acetic acid (5.8 g, 16.7 mmol) in dry dichloromethane (150 ml) was treated with oxalyl chloride (1.6 ml) and DMF (4 drops). After 1 hour at room temperature the clear solution was evaporated to dryness *in vacuo*. $\nu_{max}$ ($CHCl_3$) 3440, 1795, 1730, 1495 and 1350 $cm^{-1}$.

The acid chloride was dissolved in THF (30 ml) and added dropwise to a solution of benzyl 6,β-amino-6,α-methylthiopenicillanate (6.45 g, 18.3 mmol) and pyridine (1.6 ml) in THF (100 ml) at 0°C. The reaction was allowed to gain room temperature, stirred for 1 hour, diluted with ethyl acetate (250 ml), washed with water (5 x 100 ml), brine (100 ml), dried over magnesium sulphate, filtered, evaporated and chromatographed on silica gel eluting with 40% ethyl acetate and cyclohexane to yield 9.5 g, 84.1%, $\nu_{max}$ ($CHCl_3$) 3410, 1795, 1745, 1735, 1695, 1590 and 1350 $cm^{-1}$, δ($CDCl_3$) 0.95, 1.17, 1.36, 1.55 (6H, 4 x s, $2x2CH_3$), 2.02, 2.23 (3H, 2 x s, $5CH_3$), 4.33, 4.44 (1H,

2 x s, 3H), 5.20 (4H, s, 2 x$OCH_2$), 5.37, 5.50 (2H, 2 x s, CHCON, 5H), 5.75, 6.37 (2H, b, 2 x NH), 6.90-8.25 (13H, m, 3 x Ar).

ii) Benzyl 6,β-[D,L-2-(2-fluorophenyl)-2-(4-nitrobenzyloxycarbonylamino)acetamido]-6,α-methoxypenicillanate

A solution of 6,β-[D,L-2-(2-fluorophenyl)-2-(4-nitrobenzyloxycarbonylamino)acetamido]-6,α-methylthiopenicillante (9.4 g, 13.8 mmol) in methanol (120 ml) at room temperature was treated with mercuric acetate (4.3 g, 13.5 mmole) in methanol (60 ml). After 20 minutes the mixture was filtered, evaporated to low volume, dissolved in ethyl acetate (200 ml), washed with water (3 x 200 ml), brine (200 ml), dried over magnesium sulphate, filtered and evaporated to dryness in vacuo. Chromatographed on silica gel eluting with 40% ethyl acetate in cyclohexane to yield 4.6 g, 50.1%. $\nu_{max}$ ($CHCl_3$) 1785, 1740, 1750, 1495 and 1350cm$^{-1}$, δ ($CDCl_3$) 0.94, 1.26, 1.33, 1.36, 1.44 (6H, 4 x s, 2 x $2CH_3$), 3.25, 3.48 (3H, 2 x s, $OCH_3$), 4.35, 4.46 (1H, 2 x s, 3H), 5.20 (4H, s, 2 x $OCH_2$-), 5.56 (1H, s, 5H), 5.73, 5.88 (1H, bd, J7Hz, CHCON), 6.33, 6.47 (1H, d, J7Hz, CHNH), 6.90-8.25 (14H, m, 3 x Ar, CONH).

iii) 6,β-[D,L-2-amino-2-(2-fluorophenyl)acetamido]-6,α-methoxypenicillanic acid

A solution of benzyl 6,β-[D,L-2-fluorophenyl)-2-(4-nitrobenzyloxycarbonylamino)acetamido]-6,α-methoxy penicillante (4.2 g) in THF (10 ml), ethanol (100 ml), water (10 ml) was hydrogenated at ambient temperautre and pressures for 1 hour in the presence of 10% Pd/C (4.5 g). After filtering the solution was evaporated to low volume and the remainder freeze dried. The solid was dissolved in methanol (5 ml), filtered, precipitated with ether, filtered, washed thoroughly with ether and dried in vacuo to yield 1.5g. $\nu_{max}$ (KBr) 3350 (b), 1765, 1700, 1600, 1595, 1235 and 760cm$^{-1}$. δ($D_2O$) 0.98, 1.13, 1.22, 1.32 (6H, 4xs, $2x2CH_3$), 3.22, 3.41 (3H, 2xs, $OCH_3$), 4.07, 4.14 (1H,

2 x s, 3H), 5.4, 5.45 (2H, 2 x s, 5H, CHCON), 7.03-7.8 (4H, m, Ar).

iv) Sodium 6,β-[D,L-2-[3-[2-(4-aminosulphonylphenylamino)-4-hydroxypyrimidin-5-yl]-ureido]-2-(2-fluorophenyl)acetamido]-6,α-methoxypenicillante.

A suspension of 2-(4-aminosulphonylphenylamino)-5-amio-4 -hydroxypyrimidine (1.12 g, 4 mmol) in freshly distilled THF (100 ml) with triethylamine (0.56 ml, 4 mmol) at 0°C was treated with phosgene (4 ml of 12.5% w/w solution in toluene, 4.4 mmol). After 15 minutes the reaction was allowed to gain room temperature, stirred for 30 minutes, evaporated to half volume and added dropwise to a solution of 6,β-[D,L-2-amino-2-(2-fluorophenyl)acetamido-6α-methoxypenicillanic acid (0.8 g, 2 mmol) in 60% aqueous - THF (100 ml) at pH 7.5 with triethylamine. The pH was maintained between 7.3 and 7.8 by additions of triethylamine over 1½ hours. The THF was evaporated *in vacuo* and the aqueous residue washed with ether (2 x 100 ml), layered with ethyl acetate (150 ml) and the pH adjusted to 2.0 with 5N hydrochloric acid. The precipitated solid and the aqueous phase were discarded. The ethyl acetate phase was washed with water (2 x 100 ml), brine (100 ml), dried over magnesium sulphate, filtered and evaporated to dryness *in vacuo*. The gum was redissolved in acetone (30 ml) and 1.85N sodium 2-ethylhexanoate in 4-methylpentan-2-one added until precipitation was complete. The solid was filtered off, washed thoroughly with acetone, ether and dried *in vacuo* and chromatographed on silica gel eluting with ethyl acetate, ethanol, water (5:2:1) to yield 300 mg. $\nu_{max}$ (KBr) 3350 (b), 1765, 1655, 1590, 1530, 1335 and 1155cm$^{-1}$. δ[$(CD_3)_2SO,D_2O$] 1.09, 1.34, 1.42 (6H, 3 x s, 2 x $2CH_3$), 7.2, 7.40 (3H, 2 x s, $OCH_3$), 3.98, 4.05 (1H, 2 x s, 3H), 5.38 (1H, s, 5H),

5.73, 5.78 (1H, 2 x s, CHCON), 7.05-7.60 (4H, m, Ar) 7.64, 7.90 (4H, ABq,

J10Hz, N 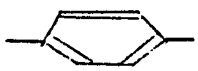 $SO_2$), 8.23 (1H, s, pyrimidin 3H).

Example 8

Sodium 6,β-[D,L-2-[3-[2-(4-aminosulphonylphenylamino)-4-hydroxy-pyrimidin-5-yl]ureido]-2-(4-fluorophenyl)-acetamido]-6,α-methoxypenicillanic.

i) Benzyl 6,β-[2-(4-fluorophenyl)-2-(4-nitrobenzyloxycarbonylamino)acetamido]-6,α-methylthiopenicillanate

Oxalyl chloride (0.95 ml) and DMF (3 drops) were added to a solution of N-(4-nitrobenzyloxycarbonyl-2-(4-fluorophenyl) glycine (3.48 g, 10 mmol) in anhydrous dichloromethane (100 ml). The solution was stirred for 1 hour than evaporated to dryness. $\nu_{max}$ (film) 1787cm$^{-1}$ (COCl). The residue was dissolved in THF (20 ml) and added dropwise to an ice bath cooled solution of benzyl 6,β-amino-6,α-methylthiopenicillante (3.87 g, 11 mmol) and pyridine (0.95 ml) in THF (100 ml). When the addition was complete the mixture was stirred at room temperature for one hour then diluted with ethyl acetate (200 ml), washed with water (5x100 ml) and brine (100 ml), dried and evaporated to a foam. The title compound was isolated by chromatography on silica gel eluting with 20% ethyl acetate in cyclohexane, 3.22 g, 47%.

ii) Benzyl 6,β-[2-(4-fluorophenyl)-2-(4-nitrobenzyloxycarbonylamino)acetamido]-6,α-methoxypenicillanate.

Benzyl 6,β-[2-(4-fluorophenyl)-2-(4-nitrobenzyloxy-carbonylamino)acetamido]-6,α-methylthiopenicillanate (3.22g) in methanol (20 ml) and ethyl acetate (20 ml) was treated with mercuric acetate (1.50g). The mixture was stirred for one hour, diluted with ethyl acetate (150 ml), and filtered. The

filtrate was washed with water (3x100 ml) and brine (100 ml), dried and evaporated to a foam which was chromatographed on silica gel eluting with 20% ethyl acetate in cyclohexane to give the title compound, 2.91 g, 93%, $\delta[(CD_3)_2CO]$ 1.10, 1.26, 1.38, 1.50 (6H, 4 x s, 2 x $2CH_3$), 3.20, 3.45 (3H, 2 x s, $OCH_3$), 4.40, 4.48 (1H, 2 x s, 3H), 5.23 (4H, s, 2 x $OCH_2$), 5.46, 5.48 (1H, 2 x s, 5H), 5.4-5.7 (1H, m, <u>CH</u>CONH), 6.9-8.3 (14H, m, Ar, $NHCO_2$), 8.68 (1H, s, CONH).

iii) <u>6,β[2-Amino-2-(4-fluorophenyl)acetamido]-6,α-methoxypenicillanic acid</u>

Benzyl 6,β-[2-(4-fluorophenyl)-2-(4-nitrobenzyloxycarbonylamino)acetamido]-6,α-methxypenicillanate (2.75g) was dissolved in THF (35 ml), diluted with water until just not cloudy then hydrogenated in the presence of 10% palladium on carbon (2.75g) for 2.5 hours. The mixture was filtered through celite and the filtrate concentrated <u>in vacuo</u>. The aqueous residue was washed with ethyl acetate (2 x 50 ml) and ether (30 ml) and freeze dried, 0.72 g, 44%, $\nu_{max}$ (KBr) 1765, 1695, 1605, 1570, 1230 and 1095cm$^{-1}$. $\delta(D_2O)$ 0.85, 1.13, 1.28, 1.37 (6H, 4 x s, 2 x $2CH_3$), 3.24, 3.46 (3H, 2 x s, $OCH_3$), 4.09, 4.16 (1H, 2 x s, 3H), 5.15, 5.22 (1H, 2 x s, <u>CH</u>CONH), 5.45, 5.49 (1H, 2 x s, 5H), 7.0-7.7 (4H, m, Ar).

iv) <u>Sodium 6,β-[D,L-2-[3-[2-(4-aminosulphonylphenylamino)-4-hydroxypyrimidin-5-yl]ureido]-2-(4-fluorophenyl)acetamido]-6,α-methoxypenicillanate</u>

A suspension of 2-(4-aminosulphonylphenylamino)-5-amino-4-hydroxypyrimidine (0.56g, 2 mmol) in freshly distilled THF (80 ml) with triethylamine (0.28 ml, 2 mmol) at 0°C was treated with phosgene (2 ml of 12.5% w/w solution in toluene, 2.2 mmol). Stirred for 1

hour, evaporated to half volume in vacuo and added dropwise to a solution of 6,β-[D,L-2-amino-2-(4-fluorophenyl)acetamido]-6,α-methoxypenicillanic acid (0.4 g, 1 mmol) in 70% aqueous THF (70 ml) at pH 7.5 with triethylamine. Allowed to warm to room temperature and stirred for 2 hours. The pH was maintained between 7.3 and 7.8 by additions of triethylamine. The mixture was washed with ether (3 x 100 ml), filtered and acidified to pH 2 with 5N hydrochloric acid. The aqueous was extracted with ethyl acetate (3 x 80 ml). The combined extracts were washed with water (3 x 50 ml), dried over magnesium sulphate, filtered and evaporated to dryness in vacuo. The gum was dissolved in acetone (20 ml) and 1.85 N sodium 2-ethylhexanoate in 4-methylpentan-2-one (0.2ml) was added. The solid was filtered off, washed thoroughly with acetone, ether and dried in vacuo to yield 260 mg, 17.1%. $\nu_{max}$ (KBr) 3350 (b), 1760, 1655, 1600, 1550, 1410 and 1155cm$^{-1}$, δ[$CD_3OD$ + $(CD_3)_2SO$] 1.03, 1.36, 1.41, 1.48 (6H, 4 x s, 2 x $2CH_3$), 3.45, 3.54 (3H, 2 x s, $OCH_3$), 3.98, 4.08 (1H, 2 x s, 3H), 5.44, 5.52, 5.58, 5.63 (2H, 4 x s, 5H, CHCON), 6.90-8.05 (8H, m, 2 x Ar), 8.29 (1H, s, pyrimidin 6H).

Example 9

Sodium 6,β-[D,L-2-[3-[2-(4-aminosulphonylphenylamino)-4-hydroxypyrimidin-5-yl]ureido]-2-(3,4-diacetoxyphenyl) acetamido]-6,α-methoxypenicillanate

i) Benzyl 6,β-[D,L-2-(3,4-diacetoxyphenyl)-2-(4-nitrobenzyloxycarbonylamino)acetamido]-6,α-methoxypenicillanate

Prepared by the method described in example 4, ii except that benzyl 6,β-[D,L,2-(3,4-diacetoxyphenyl)-2-(4-nitrobenzyloxycarbonylamino)acetamido)-6,α-methylthiopenicillanate (4.81g, 6.17 mmol) and mercuric acetate (1.97 g, 6.17 mmol) in methanol (70 ml) were used. The product was chromatogrpahed on silica gel eluting with 50% ethyl acetate in cyclohexane to yield 4.82 g, 97.2%, ($CDCl_3$) 0.89, 1.28, 1.33 (6H, 3 x s, 2 x $2CH_3$), 2.30 (6H, s, 2 x $COCH_3$), 3.23, 3.50 (3H, 2 x s, $OCH_3$), 4.40, 4.47 (1H, 2 x s, 3H), 5.28 (4H, s, 2 x $OCH_2$), 5.68 (2H, bs, 5H, CHCON), 6.5-8.4 (14H, m, 2 x CONH, 3 x Ar).

ii) 6,β-[D,L-2-Amino-2-(3,4-diacetoxyphenyl)acetamido]-6,α-methoxypenicillanate

A solution of benzyl 6,β-[D,L-2-(3,4-diacetoxyphenyl)-2-(4-nitrobenzyloxycarbonylamino)-6,α-methoxypenicillanate (4.55 g) in THF (30 ml), ethanol (100 ml) and water (20 ml) was hydrogenated at atmospheric pressure and room temperature over 10% palladium on carbon (4.6 g) for 1½ hours. The catalyst was filtered off and the solution washed with ether (3 x 150 ml). The aqueous was freeze dried to yield 2.5 g, 88%. $\nu_{max}$ (KBr) 3300 (br), 1765 (br), 1700, 1605, 1505, 1370, 1200 (br), 1105, 1010 and 900 $cm_{-1}$, $\delta(D_2O)$ 0.84, 1.13,

0091755

1.27, 1.38 (6H, 4 x s, 2 x $2CH_3$), 2.28 (6H, s, 2 x $CH_3CO$), 3.27, 3.48 (3H, 2 x s, $OCH_3$), 4.10, 4.17 (1H, 2 x s, 3H), 5.18, 5.27 (1H, 2 x s, CHCON), 5.45, 5.49 (1H, 2 x s, 5H), 7.27-7.65 (3H, m, Ar).

iii) Sodium 6,β-[D,L-2-[3-[2-(4-aminosulphonylphenylamino)-4-hydroxypyrimidin-5-yl]ureido]-2-(3,4-diacetoxyphenyl)acetamido]-6,α-methoxypenicillanate

Prepared by the method described in example (1) except that 6,β-[D,L-2-amino-(3,4-diacetoxyphenyl) acetamido]-6,α-methoxypenicillanate (0.98g, 2 mmol) was used. Chromatography on silica gel eluting with ethyl acetate: ethanol:water (5:2:1) gave 0.16 g, 19.7%. $\nu_{max}$ (KBr) 3350 (br), 1765, 1660, 1600, 1535 and 1210$cm_{-1}$, $\delta[(CD_3)_2SO+CD_3OD]$ 1.06, 1.33, 1.45 (6H, 3 x s, 2 x $2CH_3$), 2.23 (6H, s, 2 x $CH_3CO$), 3.17, 3.43 (3H, 2 x s, $OCH_3$), 4.01, 4.10 (1H, 2 x s , 3H), 5.42, 5.53, 5.61 (2H, 3 x s, CHCON, 5H), 6.90-7.95 (7H, m, 2 x Ar), 8.28 (1H, s, pyrimidin 6H).

## Biological data

Antibacterial activities of a number of compounds described (MIC ug/ml).

| ORGANISM | COMPOUND OF EXAMPLE NO. | | | | | |
|---|---|---|---|---|---|---|
| | 4 | 5 | 6 | 7 | 8 | 9 |
| E. Coli JT 425 | 1.0 | 2.5 | 8.0 | 1.0 | 16 | 1.0 |
| Ps. Aeruginosa 10662 | 10.0 | 50 | 32 | 16 | 128 | 1.0 |
| Klebsiella Aerogenes A | 0.2 | 1.0 | 0.12 | 0.25 | 8.0 | <0.06 |
| P. Mirabilis C977 | 1.0 | 2.5 | 2.0 | 1.0 | 8.0 | 2.0 |
| Staph Aureus Oxford | 10 | 25 | 16 | 16 | 64 | <128 |

A. CLAIMS

1. A compound of formula (1) or a pharmaceutically acceptable salt or an in-vivo hydrolysable ester thereof:

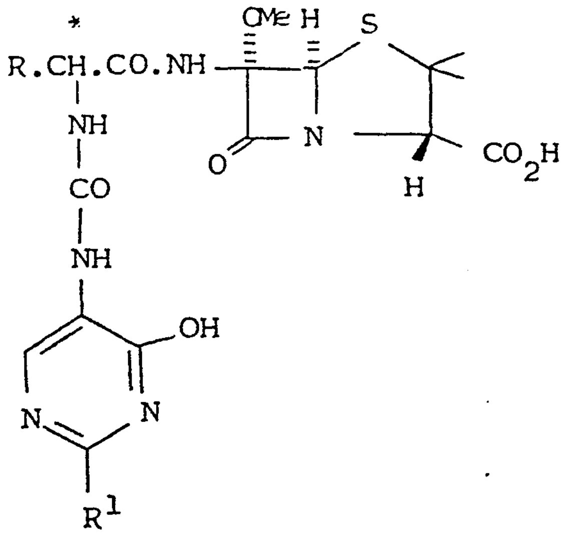

(I)

wherein R represents optionally substituted aryl or heterocyclyl; and $R^1$ represents hydrogen, an alkyl, alkenyl, cycloalkyl or cycloalkenyl group, an optionally substituted phenyl, a cyclopropyl alkyl group, a hydroxy group optionally substituted by alkyl, alkenyl, cycloalkyl, phenyl or benzyl, an optionally substituted mercapto group, an alkylsulphenyl group, a free or substituted amino group, an optionally substituted piperazino or phenyl-alkylamino group, an acylamino group or an alkyl, aralkyl or arylsulphonyl amino group.

2. A compound as claimed in claim 1 wherein the group R is phenyl, 4-hydroxyphenyl, 3,4-dihydroxy phenyl, 3,4-diacetoxy-phenyl, 2- or 3-thienyl, 2- or 3-furyl, 2-,3-, or 4-chlorophenyl, or 2-, 3-, or 4-fluorophenyl.

3. A compound as claimed in either claim 1 or claim 2 wherein $R^1$ is a substituted amino group.

4. A compound as claimed in any of claims 1 to 3 wherein $R^1$ represents an amino group substituted by an optionally substituted phenyl group.

5. A compound as claimed in claim 1 selected from:

6,β-[2-[3-[2-(4-aminosulphonylphenylamino)-4-hydroxypyrimidin-5-yl]ureido]-2-phenylacetamido]-6,α-methoxypenicillanic acid; or

6,β-[2-[3-[4-hydroxy-2-(phenylamino)pyrimidin-5-yl]ureido]-2-phenylacetamido]-6α-methoxy-penicillanic acid; or

6,β-[2-[3-[2-(4-aminosulphonylphenylamino)-4-hydroxypyrimidin-5-yl]ureido]-2-(4-hydroxyphenyl)acetamido]-6,α-methoxypenicillanic acid; or

6,β-[2-[3-[2-(4-aminosulphonylphenylamino)-4-hydroxy-pyrimidin-5-yl]ureido]-2-(thien-2-ylacetamido]-6,α-methoxypenicillanic acid; or

6,β-[2-[3-[2-(4-aminosulphonylphenylamino)-4-hydroxypyrimidin-5-yl]-ureido]-2-(furan-2-yl)acetamido]-6,α-methoxy penicillanic acid; or

6,β-[2-[3-[2-(4-aminosulphonylphenylamino)-4-hydroxypyrimidin-5-yl]ureido]-2-(2-fluorophenyl)acetamido]-6,α-methoxypenicillanic acid; or

6,β-[2-[3-[2-(4-aminosulphonylphenylamino)-4-hydroxy-pyrimidin-5-yl]ureido]-2-(4-fluorophenyl)-acetamido]-6,α-methoxypenicillanic acid; or

6,β-[2-[3-[2-(4-aminosulphonylphenylamino)-4-hydroxypyrimidin-5-yl]ureido]-2-(3,4-diacetoxyphenyl) acetamido]-6,α-methoxypenicillanic acid; or a pharmaceutically acceptable salt or in-vivo hydrolysable ester thereof.

6. A process for the preparation of a compound as claimed in claim 1 which process comprises:

A) reacting a compound of formula (II):

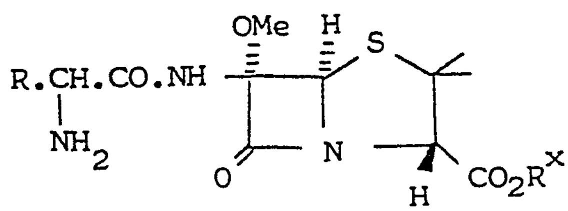

(II)

wherein the amino group is optionally substituted with a group which permits acylation to take place, R is as defined with respect to formula (I) and any reactive substituents may be protected, and $R^x$ is hydrogen or a carboxyl-blocking group, with an N-acylating derivative of an acid of formula (III):

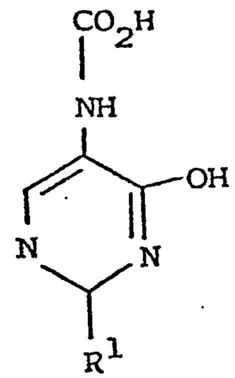

(III)

wherein $R^1$ is an defined with respect to formula (I) above, and any reactive groups may be protected;

B) reacting a compound of formula (IV) as described hereinbefore with an N-acylating derivative of an acid of formula (VI):

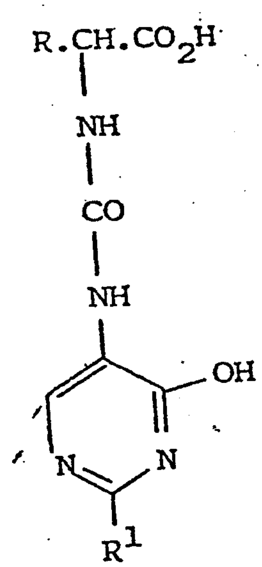

(VI)

wherein R and $R^1$ are as defined with respect to formula (I) and any reactive groups therein may be protected;

C) by reacting a compound of formula (VII):

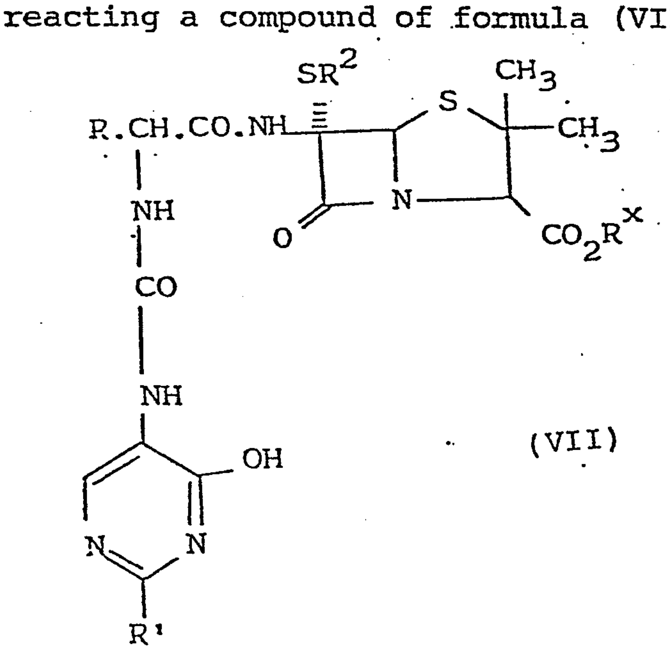

(VII)

wherein R, $R^1$ and $R^x$ are as defined above and $R^2$ is $C_{1-6}$ alkyl, aryl or benzyl; either

(i) with methanol in the presence of a metal ion, such as mercury, lead, silver, cadmium, thallium, or bismuth; or

(ii) with chlorine or bromine at -25°C to -80°C and subsequently decomposing the resultant halosulphonium halide with methanol and a base; or

D) by:

a) treating a compound of formula (IX)

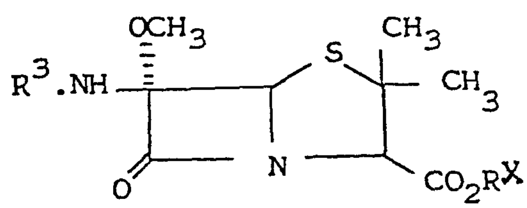

(IX)

wherein $R^x$ is a carboxyl-blocking group, and $R^3$ is an acyl group, in particular an acyl group derived from side-chain of a natural penicillin, such as benzyl penicillin or phenoxymethyl penicillin; with an agent forming an imino halide;

b) treating the imono halide with a compound to introduce a group QRf of the imino carbon atom, wherein Q is oxygen, sulphur or nitrogen and $R_f$ is an alkyl group of from 5 to 14 carbon atoms, to form an iminoether, iminothioether, or amide (when Q is O, S, or N respectively);

0091755

European Patent Office

**PARTIAL EUROPEAN SEARCH REPORT**
which under Rule 45 of the European Patent Convention shall be considered, for the purposes of subsequent proceedings, as the European search report

Application number
EP 83 30 1785

**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| Y | EP - A - 0 003 814 (DR. K. THOMAE)<br>* claims *<br>-- | 1-4,6-8 | C 07 D 499/68<br>499/70<br>A 61 K 31/43 |
| Y | EP - A - 0 006 532 (DR.K. THOMAE)<br>* claims *<br>-- | 1-4,6-8 | |
| P,X | EP - A - 0 073 454 (DR.K. THOMAE)<br>* claims *<br>------ | 1-8 | |

TECHNICAL FIELDS SEARCHED (Int. Cl. 3)

C 07 D 499/00
A 61 K 31/00

**INCOMPLETE SEARCH**

The Search Division considers that the present European patent application does not comply with the provisions of the European Patent Convention to such an extent that it is not possible to carry out a meaningful search into the state of the art on the basis of some of the claims.

Claims searched completely: 1-8
Claims searched incompletely:
Claims not searched: 9
Reason for the limitation of the search:

Method for treatment of the human or animal body by surgery or therapy (see article 52(4) of the European Patent Convention).

| Place of search | Date of completion of the search | Examiner |
|---|---|---|
| The Hague | 01-07-1983 | CHOULY |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82